# EUROPÄISCHE PATENTSCHRIFT

(11) **EP 3 522 790 B1**
(45) Veröffentlichungstag und Bekanntmachung des Hinweises auf die Patenterteilung: **02.12.2020**
(21) Anmeldenummer: 17780394.7
(22) Anmeldetag: 05.10.2017
(51) Int. Cl.: A61B 10/00, B01L 3/00, G01N 1/00

(54) **SYSTEM ZUR ASSERVATION VON HAARPROBEN**
SYSTEM FOR STORING HAIR SAMPLES
SYSTÈME POUR LA CONSERVATION D'ÉCHANTILLONS CAPILLAIRES

(30) Priorität: 06.10.2016 DE 102016118968
(43) Veröffentlichungstag der Anmeldung: 14.08.2019
(73) Patentinhaber: Eberhard Karls Universität Tübingen Medizinische Fakultät, 72074 Tübingen (DE)
(72) Erfinder: LIEBAU, Stefan, 72414 Rangendingen (DE); ACHBERGER, Kevin, 72070 Tuebingen (DE)
(74) Vertreter: Witte, Weller & Partner Patentanwälte mbB
(86) Internationale Anmeldenummer: PCT/EP2017/075336
(87) Internationale Veröffentlichungsnummer: WO 2018/065514

(56) Entgegenhaltungen:
- EP-A1- 2 284 538
- EP-A2- 1 177 769
- WO-A1-01/18239
- US-A- 5 994 149
- US-B1- 8 475 394

## Beschreibung

Die Erfindung betrifft ein System zur Asservation von Haarproben, wobei diese eine Haarwurzel und einen Haarschaft aufweisen.

Das sichere Asservieren von Haarproben, beispielsweise durch Lagern oder während eines Transports, ist in vielen Bereichen der Wissenschaft und Medizin von enormer Bedeutung. Intakte Haarproben werden für eine Vielzahl von Analysen, aber auch zur Zellkultivierung herangezogen.

Da Haarproben, ohne eine fachmännische Asservation, nach der Probenentnahme an Luft schnell beschädigt werden, wird an die Asservation von Haarproben eine Vielzahl an Herausforderungen gestellt.

Um eine Haarprobe in einer Weiterbehandlung verwenden zu können, ist es zwingend notwendig, dass die Probe vollständig intakt bleibt. Das bedeutet, dass vor allem die Haarwurzel mit äußerer Haarwurzelscheide unbeschädigt vorliegen muss, da diese häufig als Basis für die Weiterbehandlung dient. Ferner darf die Haarprobe nicht austrocknen. Auch dies verhindert eine mögliche Weiterbehandlung.

Vor einer möglichen Weiterbehandlung der Haarprobe erfolgt die Probenentnahme. Hierbei werden einem Probanden Haare entnommen. Die Haarentnahme erfolgt üblicherweise durch ausgebildetes Personal, welches in der Lage ist, die Haare mitsamt den Haarwurzeln durch geschulte Handgriffe zu entnehmen und diese für eine Weiterbehandlung vorzubereiten.

Oft liegt zwischen der Haarentnahme und dem weiteren Gebrauch der Haarprobe eine gewisse Zeitspanne, sodass die Haare zwischenzeitlich asserviert werden müssen. In einigen Fällen findet die Entnahme nicht am gleichen Ort wie die geplante Weiterbehandlung statt, sodass die Haarprobe während ihres Transports in ein entsprechendes Labor sicher asserviert werden muss. Dieser Transport gestaltet sich nach dem derzeitigen Stand der Technik ausgesprochen schwierig und zuweilen auch sehr aufwändig. Eine Möglichkeit des Transports bietet das Haarwurzel-Sammel-Kit, welches aus der EP 1 177 769 A2 bekannt ist. Hierbei wird die Haarprobe auf einen Träger aufgeklebt und somit fixiert, wobei die Haarwurzel selbst nicht aufgeklebt wird, Diese liegt stattdessen auf einem nicht-klebenden Abschnitt des Trägers. Eine zusätzliche Fixierung/Sicherung der Haarprobe kann durch eine Plastikfolie erreicht werden, die an dem Träger befestigt ist und die Haarprobe, insbesondere die Haarwurzel in Position hält.

Einige Herausforderungen, die sich während der Asservation ergeben, sind die Sicherstellung einer funktionsfähigen Haarwurzel, vorzugsweise mit äußerer Haarwurzelscheide, über die Zeit, sowie die Sicherstellung, dass die Haare nicht austrocknen. Geht die Haarwurzel während des Transportes kaputt oder trocknet das Haar aus, ist die Haarprobe für eine Weiterbehandlung unwiederbringlich zerstört und nicht mehr zu gebrauchen.

Gegenwärtig werden Haarproben in Medienbehältern, beispielsweise 15 oder 50 mL Rundbodenröhren, in einem flüssigen Medium transportiert. Hierbei kommt es häufig zu Kontaminationen der Haarprobe, da die Bauweise und Handhabung des Aufbewahrungsbehältnisses eine Sterilität über einen längeren Zeitraum nicht gewährleisten kann.

Daneben kann, aufgrund des flüssigen Aggregatzustandes des Mediums, während des gesamten Transportes, nicht sichergestellt werden, dass die Haare über die gesamte Zeit von der Flüssigkeit benetzt werden. Dies kann auftreten, wenn das Probenbehältnis während des Transports stark geschüttelt wird; häufig ist dies aber nicht zu vermeiden. Dabei kann sich die Haarprobe beispielsweise am Deckel des Transportgefäßes anlagern, sodass diese im Verlauf des weiteren Transportes nicht mehr mit dem Medium benetzt wird und austrocknet.

Häufiger jedoch läuft das Medium aus dem Behältnis aus, da die Behälter aufgrund des Schraubverschlusses für Lösungsmittel nicht dicht verschlossen sind. Sowohl das Separieren der Probe vom Medium, als auch das Auslaufen des Mediums, führt zu einem Austrocknen der Haarprobe, wodurch diese unwiederbringlich für eine Weiterbehandlung zerstört ist.

Die Verwendung von flüssigem Medium birgt ferner den Nachteil, dass bei einem Transport von insbesondere Flüssigkeiten zollrechtliche Probleme einhergehen. Ferner ist bereits das Verschicken von Flüssigkeiten in einigen Ländern nicht zulässig, sodass in bzw. aus diesen Ländern keine Haarprobe über den Postweg versendet werden kann.

Der Transport, sowie das Aufbewahren von Haarproben, werden meist bei niedrigen, möglichst konstanten Temperaturen durchgeführt, sodass der Haarprobenbehälter während des gesamten Zeitraums durchgängig gekühlt werden muss.

Ein Transport bei konstanten Temperaturen erfordert zum Teil eine ständige Kühlung, die je nach Transportweg oder der Dauer des Transportes, nur unter sehr hohem technischen Aufwand bzw. überhaupt nicht durchführbar ist. Alternativ kann der Transport von Haarproben auch bei Raumtemperatur durchgeführt werden, sofern der Transport nur für wenige Tage beansprucht.

Eine sichere Asservation gestaltet sich gemäß dem derzeitigen Stand der Technik ausgesprochen mühsam und aufwendig.

Auch vor dem Hintergrund neuer Erkenntnisse zur Gewinnung von Stammzellen aus Haarproben, besteht ein großes Bedürfnis für einen sicheren und sterilen Transport/Lagerung von Haarproben. So beschrieben Aasen et. al. (T. Aasen et al. nature biotechnology, 26, 2008, 1276 - 1284), dass die Generierung von induziert pluripotenten Stammzellen (iPS) aus Keratinozyten von Haarwurzeln, im Anschluss an die Zellkultivierung, möglich ist.

Für die iPS-Gewinnung wird die Haarprobe im Anschluss an die Haarentnahme in ein entsprechendes Ziellabor überführt. Danach wird die Probe in einem aufwendigen Verfahren, welches die Schritte der Haarplattierung, der Keratinozytkultivierung und der Reprogrammierung enthält, weiter verarbeitet. Dieses Verfahren kann sich über 35 Tage erstrecken und ist derzeitig nur durch eine qualifizierte Fachkraft durchführbar.

Diese neue Möglichkeit der Weiterbehandlung einer Haarprobe macht die sichere Asservation von Haarproben zu einem immer wichtiger werdenden Werkzeug für die wissenschaftliche und medizinische Forschung.

Vor diesem Hintergrund ist es die Aufgabe der vorliegenden Erfindung, ein neues System zur Asservation von einem eine Haarwurzel und einen Haarschaft aufweisenden Haar bereitzustellen, mit welchem die oben geschilderten Nachteile der im Stand der Technik bekannten Systeme überwunden werden können.

Erfindungsgemäß wird die Aufgabe durch ein System zur Asservation von zumindest einem eine Haarwurzel und einen Haarschaft aufweisenden Haar, wobei das System folgendes aufweist: eine Haar-Aufnahmevorrichtung, die ein Trägerplattenelement zum Ablegen des Haares und ein auf das Trägerplattenelement aufzubringendes Deckplattenelement zum teilweisen Abdecken des Haares umfasst, wobei das Trägerplattenelement ein erstes Ende, ein zweites Ende, eine Außen-Oberfläche und eine Haar-Aufnahme-Oberfläche aufweist, dadurch gekennzeichnet, dass die Haar-Aufnahme-Oberfläche einen ersten Abschnitt aufweist, der sich vom ersten Ende bis zu einem mittleren Bereich der Haar-Aufnahme Oberfläche erstreckt, und einen zweiten Abschnitt, der sich von dem mittleren Bereich bis zum zweiten Ende erstreckt, wobei der erste Abschnitt eine Aussparungen und/oder Vertiefungen aufweisende Struktur zur Aufnahme der Haarwurzel aufweist, und einen zweiten Abschnitt zur Aufnahme des Haarschafts, und dass das Deckplattenelement zum Abdecken und/oder Einklemmen des Haares auf dem Trägerplattelement derart ausgebildet und dimensioniert ist, dass es nur den zweiten Abschnitt der Haar-Aufnahme-Oberfläche des Trägerplattenelements abdeckt, gelöst.

Ferner wird die Aufgabe erfindungsgemäß durch die Verwendung des Systems zum Kultivieren von Keratinozyten und/oder Transportieren und/oder Lagern von Haarwurzel und einen Haarschaft aufweisendem Haar, gelöst.

Mit dem erfindungsgemäßen System ist die sichere Asservation von Haaren, welche eine Haarwurzel und einen Haarschaft aufweisen, über einen längeren Zeitraum bei Raumtemperatur möglich. Dabei werden die einzelnen Haare zwischen einem Trägerplattenelement und einem Deckplattenelement abgedeckt und/oder eingeklemmt. Hierdurch liegen die Haare definiert und fixiert vor. Ein Verrutschen der Haare wird mit dem erfindungsgemäßen System vermieden, da das Deckplattenelement zumindest einen Teil des Haarschaftes einklemmt. Die Haarwurzel, vorzugsweise mit äußerer Haarwurzelscheide, liegt dagegen sicher auf dem Trägerplattenelement auf.

Ein Verrutschen der Haarprobe kann erfindungsgemäß bereits durch vorheriges Anfeuchten der Haare oder durch eine polare/elektrostatische Oberflächenbehandlung verhindert werden. Ein angefeuchtetes Haar bzw. ein aufgeladenes Haar kann auf der Haar-Aufnahme-Oberfläche bereits von sich aus kleben.

Erfindungsgemäß wird unter "Haarschaft" der Bereich des Haares verstanden, der aus der Haut herausragt und mit bloßem Auge sichtbar ist. Der in der Haut befindliche Abschnitt wird erfindungsgemäß als "Haarwurzel" verstanden. Die Haarwurzel umfasst die äußere Haarwurzelscheide mit den dort befindlichen, teilungsfähigen HaarKeratinozyten.

Unter "Asservation" wird erfindungsgemäß das Abdecken bzw. Einklemmen und Aufbewahren von Haarproben verstanden, wobei die Asservation auch während eines Transports möglich ist.

Vorliegend wird unter "Haarprobe" eine Probe von zumindest einem Haar verstanden, welches sowohl einen Haarschaft als auch eine Haarwurzel aufweist. Die "Haarprobe" stammt erfindungsgemäß bevorzugt von einem Probanden, der vorzugsweise ein Säugetier, vorzugsweise ein Mensch ist, sodass sich verschiedene Haarproben in dem jeweiligen Spender unterscheiden.

Erfindungsgemäß weist die Haar-Aufnahme-Oberfläche des Trägerplattenelements zumindest zwei Abschnitte auf, welche sich dabei in der Oberflächenstruktur unterscheiden können; einen ersten Abschnitt und einen zweiten, die in einem mittleren Bereich aneinander grenzen, und die gemäß einer Ausführungsform jeweils die Hälfte der Fläche einnehmen. Es versteht sich, dass diese hälftige Einteilung lediglich beispielhaft ist, und die Einteilung in den ersten und den zweiten Abschnitt auch anderweitig prozentual aufgeteilt sein kann, von zwischen 10% der Haar-Aufnahme-Oberfläche des Trägerplattenelements für den ersten Abschnitt und 90% für den zweiten Abschnitt, bis ca. 90% für den ersten Abschnitt und 10% für den zweiten Abschnitt. Bevorzugte Flächenverteilungen liegen für das Flächenverhältnis des ersten Abschnitts zum zweiten Abschnitt bei ca. 30:70, ca. 40:60; ca. 50:50, ca. 60:40, ca. 30:70, wobei es sich versteht, dass diese Bereichsangaben nicht exakt vorliegen müssen.

Gemäß einer weiteren Ausführungsform, nimmt der erste Abschnitt ein Drittel und der zweite entsprechend zwei Drittel der Fläche ein, oder umgekehrt, sodass der erste Abschnitt zwei Drittel der Fläche entspricht und der zweite Abschnitt einem Drittel. Entsprechendes gilt für die Flächeneinteilung in Vierteln, Fünfteln etc.

Während der erste Abschnitt eine Aussparungen und/oder Vertiefungen aufweisende Struktur besitzt, weist der zweite Abschnitt bevorzugt eine glatte Oberfläche auf. Die glatte Oberfläche des zweiten Abschnittes ermöglicht ein besseres Abdecken und/oder Einklemmen des Haares durch das Deckplattenelement als beispielsweise auf dem ersten Abschnitt. Der mittlere Bereich kann je nach Ausführungsform eine Aussparungen und/oder Vertiefungen aufweisende Struktur aufweisen und/oder alternativ eine glatten Oberfläche aufweisen. Andere Oberflächenstrukturen des mittleren Bereichs und des zweiten Abschnitts sind erfindungsgemäß möglich.

Gemäß einer bevorzugten Ausführungsform weist das Trägerplattenelement eine längliche Form auf. Bevorzugte Maße sind gemäß einer weiteren Ausführungsform der Länge entsprechend ca. 50 bis 150 mm, insbesondere ca. 65 bis 85 mm, der Breite entsprechend ca. 15 bis 45 mm, insbesondere 20 bis 30 mm und der Höhe entsprechend ca. < 2,5 mm, insbesondere ≤ 1 mm. In einer bevorzugten Ausführungsform weist das Trägerplattenelement beispielsweise die Maße 76 mm (Länge), 26 mm (Breite) und 1 mm (Höhe) auf. Entsprechend ist, gemäß einer bevorzugten Ausführungsform, das Deckplattenelement im Wesentlichen länglich oder quadratisch. Bevorzugte Maße sind gemäß einer weiteren Ausführungsform der Länge entsprechend ca. 25 bis 75 mm, insbesondere ca.35 bis 40 mm, der Breite entsprechend ca. 15 bis 45 mm, insbesondere 20 bis 30 mm und der Höhe entsprechend < 3 mm, insbesondere < 1 mm. Somit sind das Trägerplattenelement und das Deckplattenelement im Wesentlichen rechteckig, vorzugsweise mit zwei schmaleren/kürzeren Seiten und zwei längeren Seiten. Erfindungsgemäß kann das Trägerplattenelement sowie das Deckplattenelement weitere Formen wie beispielsweise ovale, runde oder geschwungene Formen aufweisen.

Gemäß einer bevorzugten Ausführungsform des erfindungsgemäßen Systems wird das Deckplattenelement mittels eines Befestigungsmittels auf dem Trägerplattenelement befestigt, zum Abdecken und/oder Einklemmen von zumindest einem eine Haarwurzel und einen Haarschaft aufweisenden Haar.

Unter "Befestigungsmittel" wird dabei erfindungsgemäß jedes Mittel verstanden, mit dem die beiden Plattenelemente befestigt werden können, wobei bevorzugt ist, wenn sich das Befestigungsmittel reversibel anbringen lässt, sodass die Haarprobe bei Bedarf neu angeordnet und wieder eingeklemmt werden kann. Ein mögliches Befestigungsmittel stellt beispielsweise eine Klemme (z.B. Clip System) dar, welche das Trägerplattenelement und das Deckplattenelement umschließt. Alternativ können die beiden Platten erfindungsgemäß miteinander verklebt werden, sodass die Haare eingeklemmt in der Haar-Aufnahmevorrichtung vorliegen. Ferner können die beiden Plattenelemente auch aufgrund ihrer Bauweise miteinander verklemmt werden; beispielsweise durch passendes Ineinanderschieben bzw. ineinanderstecken. Ferner ist ein Verschrauben der Platten möglich.

Diese Ausführungsform hat den Vorteil, dass eine bessere Fixierung des Haarschafts innerhalb der Haar-Aufnahmevorrichtung durch das Deckplattenelement erreicht wird. Ein Verrutschen der Platten, sowie der Haarprobe, kann durch diese Ausführungsform unterbunden werden.

In einer weiteren Ausführungsform der Erfindung ist das erste Ende des Trägerplattenelements, im Vergleich zu dem restlichen Trägerplattenelement, erhöht.

Erhöhung bedeutet dabei, dass das erste Ende eine größere Höhe aufweist, im vergleich zum zweiten Ende des Trägerplattenelements. Die Erhöhung kann sich erfindungsgemäß stufenförmig ergeben, sodass am ersten Ende ein bevorzugt schmaler Balken auf dem Trägerplattenelement aufliegt. Ebenso kann sich die Erhöhung erfindungsgemäß linear ergeben, sodass der erste Abschnitt im Bereich zum ersten Ende kontinuierlich an Höhe zunimmt. Diese linear ansteigende Erhöhung kann sich über den ganzen erste Bereich erstrecken, oder lediglich zum ersten Ende hin.

Diese Ausführungsform hat den Vorteil, dass ein Verschieben der Haarwurzel, welche sich auf dem ersten Abschnitt der Haar-Aufnahme-Oberfläche befindet, über das erste Ende hinüber unterbunden werden kann. Zudem schützt die Erhöhung des Trägerplattenelements, die Haarwurzel vor äußeren Einflüssen, ausgehend vom ersten Ende des Trägerplattenelements.

Gemäß einer bevorzugten Ausführungsform der Erfindung ist die Aussparungen und/oder Vertiefungen aufweisende Struktur des ersten Abschnittes des Trägerplattenelements zwischen der Haar-Aufnahme-Oberfläche und der Außen-Oberfläche ganz durchlässig.

Diese Ausführungsform hat den Vorteil, dass die Haarwurzel, welche sich auf dem ersten Abschnitt der Haar-Aufnahme-Oberfläche befindet, von allen Seiten zugänglich ist und zugleich an ihrem Haarschaft fixiert auf der Haar-Aufnahme-Oberfläche vorliegt.

Gemäß einer weiteren bevorzugten Ausführungsform weist das System ferner ein Transportbehältnis und/oder ein Lagerungsbehältnis mit zumindest einer Öffnung zum Einschieben zumindest einer Haar-Aufnahmevorrichtung auf, wobei die Öffnung des Transportbehältnisses und des Lagerungsbehältnisses im Wesentlichen eine gleiche Höhe und Breite entsprechend des ersten Endes der Haar-Aufnahme-Oberfläche aufweist, dass die Haar-Aufnahmevorrichtung, mit ihrem ersten Abschnitt, zumindest teilweise in die Öffnung des Transportbehältnisses einschiebbar ist.

Diese Ausführungsform hat den Vorteil, dass die Asservation von Haarproben innerhalb eines Transport- oder eines Lagerungsbehältnis durchgeführt werden kann. Hierbei kann vor allem die Haarwurzel vor äußeren Einflüssen geschützt werden. Zudem ist durch diese Ausführungsform ein steriles Transportieren bzw. Lagern von Haarproben möglich.

Gemäß einer bevorzugten Ausführungsform weist das Transport- bzw. das Lagerungsbehältnis eine längliche Form auf. Bevorzugt, wird die Haar-Aufnahmevorrichtung mit einer der schmalen Seiten in das Transport- bzw. Lagerungsbehältnis eingeschoben. Somit entspricht die Öffnung in einer weiteren Ausführungsform der bevorzugten Maße entsprechend der Länge ca.16 bis 46 mm, insbesondere ca. 21 bis 31 mm und entsprechend in der Höhe ca. < 4 mm, insbesondere < 2 mm.

Das Transportbehältnis entspricht in seinen Ausmaßen im Wesentlichen den Ausmaßen des Trägerplattenelements, sodass ein platzsparender Transport von Haarproben erfolgen kann. Bevorzugte Maße sind gemäß einer weiteren Ausführungsform der Länge entsprechend ca. 40 bis 100 mm, insbesondere ca 50 bis 70 mm, der Breite entsprechend ca. 25 bis 70 mm, insbesondere ca. 35 bis 45 mm und in der Höhe entsprechend ca. 10 mm bis 40 mm, insbesondere 15 bis 30 mm.

Das Lagerungsbehältnis weist in einer bevorzugten Ausführungsform mehrere Öffnungen zur Aufnahme von Trägerplattenelementen auf. Demnach ist es möglich mehrere Trägerplattenelemente parallel zu lagern. Dies ermöglicht eine platzsparende Lagerung, Dem Fachmann wird dabei klar sein, welche Maße des Lagerungsbehältnisses besonders vorteilhaft sind. Außerdem wird dieser einschätzen können, welche Anzahl der Öffnungen für die Trägerplattenelemente im Lagerungsbehältnis sinnvoll erscheint.

In einer weiteren Ausführungsform des Systems weist das Transportbehältnis feuchtigkeitsspendende Gele auf.

Erfindungsgemäß wird unter "Gel" ein feindisperses System verstanden, analog zu einem viskoelastischen Fluid. Die Fluideigenschaften des erfindungsgemäßen Gels liegen zwischen der einer idealen Flüssigkeit und der eines idealen Feststoffs.

Diese Ausführungsform bietet den Vorteil, dass feuchtigkeitsspendende Gele ein Austrocknen der Haarwurzel, sowie des Haarschafts verhindern können. Aufgrund des erfindungsgemäßen Aufbaus des Transportbehältnisses und der darin befindlichen Haar-Aufnahmevorrichtung kann die Haarwurzel stets mit dem feuchtigkeitsspendenden Gel, welches sich bevorzugt auf der Außen-Oberfläche befindet, benetzt werden, ohne im Gel zu ertrinken. Somit ist die Haarwurzel zum einen mit dem feuchtigkeitsspendendem Gel benetzt, zum andere jedoch an Luft gelagert.

Aufgrund der Konsistenz des Gels ermöglicht diese Ausführungsform, dass das Gel im Transportbehältnis sicher verbleibt. Ein Auslaufen, wie beispielsweise bei Flüssigkeiten, wird vermieden.

Das Transport- bzw. das Lagerungsbehältnis können erfindungsgemäß zumindest annähernd die gleichen Ausmaße aufweisen, wie die Haar-Aufnahmevorrichtung. Hierdurch ergibt sich der Vorteil einer besonders platzsparenden Asservation von Haaren.

Erfindungsgemäß kann das Transport- bzw. das Lagerungsbehältnis mehrere Öffnungen für mehrere Haar-Aufnahmevorrichtungen aufweisen. Hierdurch sind der Transport und die Lagerung mehrerer Haarproben in nur einem Behältnis möglich. Dabei können Material- sowie Lagerungs- und Transportkosten eingespart werden.

Sofern die Lagerung unter Kühlung stattfindet, hat diese Ausführungsform den Vorteil, dass Kosten, die durch die Kühlung verursacht werden, gering gehalten werden, da mehrere Haar-Aufnahmevorrichtungen nebeneinander sowie hintereinander im Lagerungsbehältnis eingelagert werden können. Hierdurch ergibt sich eine besonders platzsparende Lagerung von Haarproben.

Gemäß einer bevorzugten Ausgestaltung des erfindungsgemäßen Systems weist dieses ferner ein Kultivierungsbehältnis mit zumindest einer ersten Öffnung zum Einschieben zumindest einer Haar-Aufnahmevorrichtung auf, wobei die erste Öffnung des Kultivierungsbehältnisses im Wesentlichen eine gleiche Höhe und Breite entsprechend des ersten Endes der Haar-Aufnahme-Oberfläche aufweist, dass die Haar-Aufnahmevorrichtung mit ihrem ersten Abschnitt zumindest teilweise in die Öffnung des Kultivierungsbehältnisses einschiebbar ist, sowie einer zweite Öffnung zum Austausch und/oder Zugabe von Kultivierungsmedien.

Diese Ausführungsform hat den Vorteil, dass ausgehend von zumindest einer Haarwurzel Keratinozyten kultiviert werden können. Die gewonnenen Keratinozyten können wie jede andere proliferierenden Zellart für Zwecke genetischer Analysen verwendet werden.

Aufgrund des einfachen Handlings kann das erfindungsgemäße System von jeder Person ohne Schulung genutzt werden um Keratinozyten zu kultivieren.

In einer weiteren Ausführungsform kann das System zur Generierung von induziert pluripotenten Stammzellen (iPS) verwendet werden. Die Generierung von induziert pluripotenten Stammzellen erfolgt dabei auf Basis der Keratinozyten, wobei diese ausgehend von den Haarwurzeln kultiviert wurden. Mit dieser Ausführungsform ist es möglich, sowohl die Kultivierung als auch die Reprogrammierung innerhalb eines Gefäßes ohne Umplattierung durchführen zu können.

Gemäß dieser Ausführungsform kann das Verfahren der iPS-Gewinnung aus Haarkeratinozyten für etablierte Labore vereinfacht, wiederholbar und kostengünstig gestaltet werden.

Das erfindungsgemäße System bietet ferner die Möglichkeit, in großem Stil für geringen Kostenaufwand individualisierte Zellen zu entnehmen und als Biobank auch ohne die Notwendigkeit der direkten Reprogrammierung zu konservieren. Im Zuge dessen kann ein komplett automatisiertes Barcodesystem eingeführt werden, dass eine anonymisierte Probensammlung gewährleistet und datenschutztechnischen Anforderungen gerecht werden kann.

Erfindungsgemäß weist das Kultivierungsbehältnis zumindest eine Öffnung zum Einschieben einer Haar-Aufnahmevorrichtung auf. Erfindungsgemäß kann das Kultivierungsbehältnis auch mehrere Öffnungen zum Einschieben mehrerer Haar-Aufnahmevorrichtungen aufweisen. Hierdurch ist die Kultivierung verschiedener Haarproben auf verschiedenen Haar-Aufnahmevorrichtungen parallel in einem Kultivierungsbehältnis möglich. Diese Ausführungsform bietet vor allem den Vorteil der Zeit- und Kostenersparnis.

In einer bevorzugten Ausführungsform weist das Kultivierungsbehältnis eine quaderförmige Form auf. In einer weiteren Ausführungsform entspricht die Form des Kultivierungsbehältnisses einer Kultivierungsflaschenform, beispielsweise einer T25-Zellkulturflasche. Das Kultivierungsbehältnis weist ferner bevorzugt eine seitliche Öffnung für die Haar-Aufnahmevorrichtung auf, welche die bevorzugten Maße entsprechend der Länge von ca. 16 bis 46 mm, insbesondere ca. 21 bis 21 mm und entsprechend der Höhe von ca. < 4 mm, insbesondere <2 mm aufweist.

In einer bevorzugten Ausführungsform entspricht die zweite Öffnung, zum Austausch und/oder Zugabe von Kultivierungsmedien einer Flaschenöffnung, einem Schraubverschluss oder einem Deckel.

Ferner kann das Kultivierungsbehältnis mehrere Öffnungen zum Austausch und/oder Zugabe von Kultivierungsmedien aufweisen. Hierdurch kann die Kultivierung von Keratinozyten und/oder die Reprogrammierung zu iPS Zellen automatisiert durchgeführt werden, wodurch sich eine Zeit- und Kostenersparnis ergibt.

Erfindungsgemäß können die Haar-Aufnahmevorrichtung sowie das Kultivierungsbehältnis aus sämtlichen Materialien hergestellt sein, welche eine ausreichende Adhärenz der Zellen an der Kultivierungsoberfläche ermöglichen.

Die Kultivierungsoberfläche kann erfindungsgemäß die Haar-Aufnahmeoberfläche sowie die Außen-Oberfläche des Trägerplattenelements darstellen.

Gemäß einer weiteren Ausführungsform sind das Trägerplattenelement, das Deckplattenelement, das Transportbehältnis, das Kultivierungsbehältnis und/oder das Lagerungsbehältnis aus einem Material, ausgewählt aus der Gruppe bestehend aus Polypropylen, Polyethylen, Polysulfon, Teflon, FEP, Teflon PFA, Polystyrol, Polycarbonat, Styrol, Acrylonitril, Acryl, Glas und Mischungen davon, hergestellt.

Diese Ausführungsform hat den Vorteil, dass die Materialien chemisch inert sind. Diese Materialien sind stabil gegenüber verschiedenen Lösungsmitteln, sodass ein Herauslösen von Substanzen des Materials zumindest größtenteils ausgeschlossen ist.

Einige dieser Materialien bieten den Vorteil, dass sie elektrostatische Eigenschaften aufweisen. Aufgrund dieser Eigenschaften ist es möglich, dass das Haar vor allem durch die elektrostatische Anziehung auf der Haar-Aufnahme-Oberfläche fixiert ist. Ein Verschieben des Haares, insbesondere der Haarwurzel, kann somit zumindest teilweise verhindert werden.

In einer weiteren Ausführungsform weist das Kultivierungsbehältnis Kultivierungsmedien ausgewählt aus der Gruppe bestehend aus Verbindungen zur Verbesserung der Zelladhäsion, Nährmedien, Keratinozytenmedien, Fibroblasten-Wachstumsfaktoren, Peptidasen, Antibiotika, Antimykotika oder Mischungen dieser auf.

Diese Ausführungsform bietet den Vorteil, dass durch das Vorhandensein des Kultivierungsmediums eine Kultivierung der Keratinozyten ausgehend von der Haarwurzel möglich ist. Diese Ausführungsform bietet den Vorteil, dass eine Kultivierung der Keratinozyten auch durch ungeschultes Personal durchgeführt werden kann.

Die Erfindung betrifft somit ein System zur Asservation von Haaren, das zumindest die erfindungsgemäße Haar-Aufnahmevorrichtung aufweist, sowie gegebenenfalls das erfindungsgemäßen Transport- oder Lagerungsbehältnis, sowie gegebenenfalls - zusätzlich oder alternativ- das Kultivierungsbehältnis. Dadurch wird ein praktikables Baukastensystem bereitgestellt, das sowohl die Handhabung, als auch die Kultivierung der Haarzellen deutlich vereinfacht.

Weitere Vorteile ergeben sich aus den Figuren der nachstehenden Beschreibung bevorzugter Ausführungsbeispiele.

Es versteht sich, dass die vorstehend genannten und die nachstehend noch zu erläuternden Merkmale nicht nur in der jeweilig angegebenen Kombination, sondern auch in anderen Kombinationen oder in Alleinstellung verwendbar sind, ohne den Rahmen der vorliegenden Erfindung zu verlassen.

Ausführungsbeispiele der Erfindung sind in den Zeichnungen dargestellt und werden nachfolgend erläutert. Es zeigen:
- Figur 1A: eine schematische Darstellung einer ersten Ausführungsform des Systems zur Asservation von zumindest einem eine Haarwurzel und einen Haarschaft aufweisenden Haar;
- Figur 1B: eine schematische Darstellung einer zweiten Ausführungsform des Systems zur Asservation von zumindest einem eine Haarwurzel und einen Haarschaft aufweisenden Haar;
- Figur 2: eine schematische Darstellung einer dritten Ausführungsform des Systems zur Asservation von zumindest einem eine Haarwurzel und einen Haarschaft aufweisenden Haar, wobei neben der Haar-Aufnahmevorrichtung ein Transportbehältnis gezeigt ist;
- Figur 3: eine schematische Darstellung einer vierten Ausführungsform des Systems zur Asservation von zumindest einem eine Haarwurzel und einen Haarschaft aufweisenden Haar, wobei neben der Haar-Aufnahmevorrichtung ein Kultivierungsbehältnis gezeigt ist; und
- Figur 4: eine schematische Darstellung einer fünften Ausführungsform des Systems zur Asservation von zumindest einem eine Haarwurzel und einen Haarschaft aufweisenden Haar, wobei neben der Haar-Aufnahmevorrichtung ein Lagerungsbehältnis gezeigt ist.

Funktionsäquivalente Elemente werden in allen Figuren, auch bei unterschiedlichen Ausführungsformen, mit den gleichen Bezugszeichen gekennzeichnet.

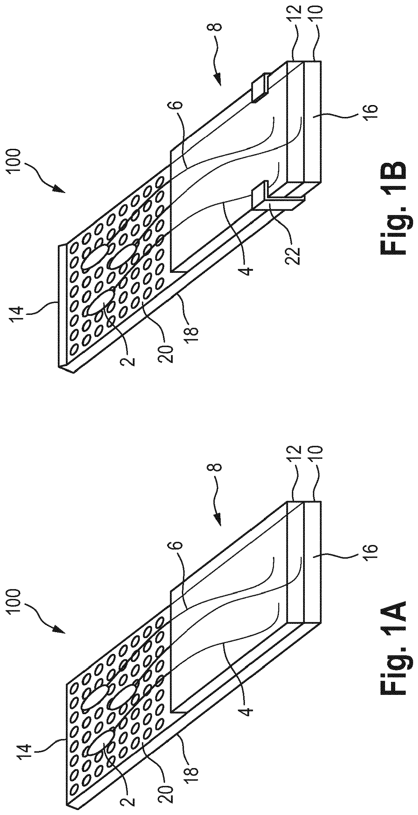

Fig. 1A zeigt eine schematische Darstellung einer ersten Ausführungsform des Systems 100 zur Asservation von zumindest einem eine Haarwurzel 2 und einen Haarschaft 4 aufweisenden Haar 6. Zu erkennen ist eine Haar-Aufnahmevorrichtung 8, welche aus einem Trägerplattenelement 10 und einem Deckplattenelement 12 aufgebaut ist. Die Haar-Aufnahmevorrichtung 8 ist dabei derart beschaffen, dass ein Haar 6, welches eine Haarwurzel 2 und einen Haarschaft 4 aufweist, zwischen dem Trägerplattenelement 10 und dem Deckplattenelement 12 abgedeckt ist. Abgedeckt ist dabei ein Großteil des Haarschaftes 4, wobei der Rest des Haarschafts 4 und die Haarwurzel 2 auf der Haar-Aufnahme-Oberfläche 20 des Trägerplattenelements 10 aufliegen.

Neben der dargestellten rechteckigen Form der Haar-Aufnahmevorrichtung 8 ist erfindungsgemäß jede weitere Form möglich, beispielsweise rechteckig, oval oder rund. Je nach Ausführungsform kann es vorteilhaft sein, dass die Haar-Aufnahmevorrichtung 8 T-förmig geformt ist.

Wie aus der Fig. 1A ersichtlich ist, weist das Trägerplattenelement 10 ein erstes Ende 14 und ein zweites Ende 16 auf. Neben der Außen-Oberfläche 18, welche in dieser Figur die untere Oberfläche bildet, weist das Trägerplattenelement 10 zudem eine Haar-Aufnahme-Oberfläche 20 auf. Diese Haar-Aufnahme-Oberfläche 20 weist einen ersten Abschnitt auf, der sich vom ersten Ende 14 bis zu einem mittleren Bereich der Haar-Aufnahme-Oberfläche 20 erstreckt. Dieser erste Abschnitt weist eine Aussparungen und/oder Vertiefungen aufweisende Struktur zur Aufnahme der Haarwurzel 2 auf. Der zweite Abschnitt erstreckt sich vom mittleren Bereich bis zum zweiten Ende 16, wobei dieser eine glatte Oberfläche aufweist. Die glatte Oberfläche ist dabei besonders vorteilhaft für das Abdecken der Haare mittels des Deckplattenelements 12.

In einer bevorzugten Ausführungsform ist das System 100, zur Asservation von Haaren 6, für einen einmaligen Gebrauch zu verwenden. Dabei kann das System 100 vor dem Gebrauch steril verpackt sein.

Fig. 1B zeigt eine zweite Ausführungsform des Systems 100 zur Asservation von zumindest einem eine Haarwurzel 2 und einen Haarschaft 4 aufweisenden Haar 6. Diese schematische Darstellung beinhaltet die schematische Darstellung aus Fig. 1A mit den dort beschriebenen Merkmalen.

In Fig. 1B ist ferner das Deckplattenelement 12 mit dem Trägerplattenelement 10 durch ein Befestigungsmittel 22 befestigt. Mit dem Befestigungsmittel 22 soll erreicht werden, dass eine Verschiebung des Haares 6 mit dem Haarschaft 4 im zweiten Abschnitt möglichst verhindert werden soll. Ferner sind erfindungsgemäß jede Form von Befestigungsmittel 22 möglich, welche die Trägerplattenelemente 10 und Deckplattenelemente 12 zusammen halten. Beispielsweise können die Platten verklebt, verschraubt oder verklemmt werden.

Ferner weist die Haar-Aufnahmevorrichtung 8 und deren Trägerplattenelement 10 eine Erhöhung am ersten Ende 14 auf. Erhöhung bedeutet dabei, dass ein Querschnitt des ersten Endes des ersten Abschnitts bevorzugt den Maßen entsprechend der Höhe von <5 bis < 1.5 mm, insbesondere <2 bis 1,5 mm entspricht. Ein Querschnitt des ersten und des zweiten Endes der Haar-Aufnahmevorrichtung unterscheiden sich demnach in ihrer Höhe nicht aber in der Breite und Länge. Die Erhöhung kann sich stufenweise oder linear ergeben.

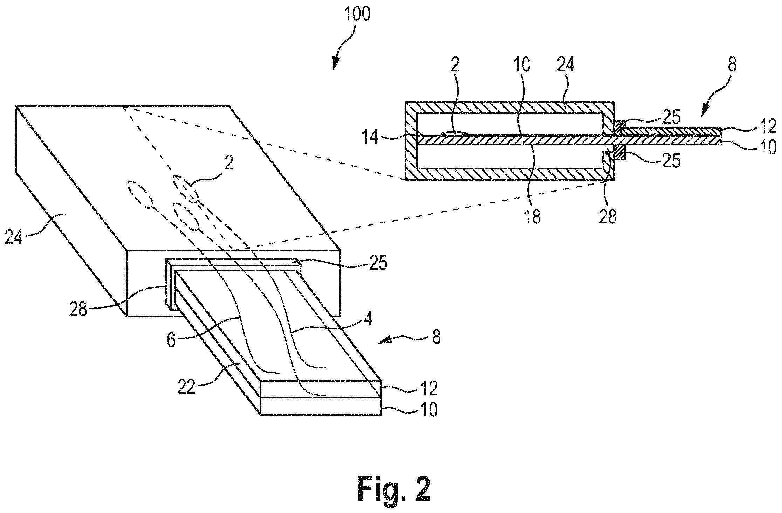

In Fig. 2 ist eine weitere Ausführungsform des Systems 100 zur Asservation von zumindest einem eine Haarwurzel 2 und einen Haarschaft 4 aufweisenden Haar 6 gezeigt, wobei neben der Haar-Aufnahmevorrichtung 8 ein Transportbehältnisses 24 gezeigt ist. Neben der perspektivischen Darstellung des Systems 100 ist auch ein Querschnitt des Systems 100 in dieser Figur erkennbar. Die schematische Darstellung der Fig. 2 beinhaltet ferner die schematische Darstellung der Haar-Aufnahmevorrichtung 8 aus Fig. 1A mit den dort beschriebenen Merkmalen.

Fig. 2 zeigt ferner die Haar-Aufnahmevorrichtung 8, welche durch die Öffnung 28 des Transportbehältnisses 24 geschoben ist und sich dadurch zum Teil im Transportbehältnis 24 befindet. Die Öffnung 28 ist dabei derart beschaffen, dass sie im Wesentlichen eine gleiche Höhe und Breite entsprechend des ersten Endes 14 der Haar-Aufnahme-Oberfläche 20 aufweist, sodass die Haar-Aufnahmevorrichtung 8 mit ihrem ersten Abschnitt zumindest teilweise in die Öffnung 28 des Transportbehältnisses 24 einschiebbar ist. Das zum Teil freiliegende Haar, welches nicht durch das Deckplattenelement 12 abgedeckt ist, ist somit im Transportbehältnis 24 gelagert.

Das Transportbehältnis 24 weist bevorzugt ein feuchtigkeitsspendendes Gel auf (nicht gezeigt). Dieses feuchtigkeitsspendende Gel hat den Vorteil, dass ein Austrocknen des Haares 6 mitsamt der Haarwurzel 2 und dem Haarschaft 4 während des Transports verhindert werden kann. Neben dem feuchtigkeitsspendenden Gel können sich andere feuchtigkeitsspendende Materialien und/oder Flüssigkeiten befinden, die das austrocknen der Haare 6 verhindern können. Ein Vorteil des feuchtigkeitsspendenden Gels ist, dass Aufgrund der Zusammensetzung des Gels der Transport der Haare 6 auch bei Raumtemperatur stattfinden kann. Ferner werden die Nachteile, die sich durch den Transport mit Flüssigkeiten ergeben, aufgrund der Konsistenz des Gels überwunden.

Die Öffnung 28 des Transportbehältnisses 24 kann ferner eine Dichtung 25 aufweisen. Diese Dichtung 25 kann das Austrocknen der Haare als auch das Auslaufen der darin befindlichen Gele verhindern. Als Dichtung 25 kann erfindungsgemäß jede Form der Abdichtung verwendet werden. Beispielsweise kann eine Dichtungsart verwendet werden, mit der ein Einclippen der Haar-Aufnahme-Oberfläche erlaubt wird. Dabei kann eine zugfeste Verbindung zwischen Transportbehältnis und Haar-Aufnahme-Oberfläche hergestellt werden, die durch einen Freisetzungs-Mechanismus, beispielsweise durch seitliches Drücken, gelöst werden kann.

Wie in Fig. 2 gezeigt, weist das Transportbehältnis 24 annähernd die gleichen Ausmaße auf, wie die Haar-Aufnahmevorrichtung. Dies bietet den Vorteil, dass das Transportbehältnis 24 zusammen mit der Haar-Aufnahmevorrichtung 8 relativ klein ist, was einen erleichterten Transport möglich macht.

In dem Querschnitt des Systems 100 ist in Fig. 2 ferner gezeigt, dass die Öffnung 28 zum Einschieben der Haar-Aufnahmevorrichtung 8 derart im Transportbehältnis 24 angebracht ist, dass, wenn sich die Haar-Aufnahmevorrichtung 8 im Transportbehältnis 24 befindet, sowohl auf der Seite der Haar-Aufnahme-Oberfläche 20 als auch auf der Seite der Außen-Oberfläche 18 des Trägerplattenelements 10 sich innerhalb des Transportbehältnisses 24 zwei Bereiche ergeben. Dabei wird ein oberer Bereich als auch ein unterer Bereich gebildet, welcher durch die Haar-Aufnahmevorrichtung 8 unterbrochen ist.

In einer Ausführungsform kann sich das feuchtigkeitsspendende Gel im oberen Bereich befinden, während im unteren Bereich eine Luftzirkulation möglich ist. Das feuchtigkeitsspendende Gel kann ferner im unteren Bereich des Transportbehältnisses 24 gelagert sein, sodass die Luftzirkulation im oberen Bereich stattfinden kann.

In Fig. 2 ist gezeigt, dass die Haar-Aufnahmevorrichtung 8 mit der Haar-Aufnahme-Oberfläche 20 nach oben in das Transportbehältnis 24 eingeschoben ist. Ferner ist es möglich, die Haar-Aufnahmevorrichtung 8 umgekehrt in das Transportbehältnis 24 zu schieben.

In Fig. 2 weist das Transportbehältnis 24 eine Öffnung 28 für die Haar-Aufnahmevorrichtung 8 auf. In einer bevorzugten Ausführungsform (nicht gezeigt) ist es möglich, dass das Transportbehältnis 24 mehrere Öffnungen 28 zum Transportieren mehrerer Haar-Aufnahmevorrichtungen 8 aufweist. Diese Haar-Aufnahmevorrichtungen 8 können nebeneinander als auch hintereinander angeordnet sein. Hierdurch ist der Transport mehrerer Haar-Aufnahmevorrichtungen 8 besonders platzsparend und kostengünstig möglich.

In einer weiteren Ausführungsform (nicht gezeigt) ist der erste Abschnitt des Trägerplattenelements 10, welches eine Aussparungen und/oder Vertiefungen aufweisende Struktur aufweist, zwischen der Haar-Aufnahme-Oberfläche 20 und der Außen-Oberfläche 18 ganz durchlässig. Diese Ausführungsform bietet den Vorteil, dass sofern sich ein feuchtigkeitsspendendes Gel im Transportbehältnis 24 befindet, die Haarwurzel 2 zum Teil vom Gel benetzt werden kann und zum anderen das Haar 6 an der Luft atmen lässt.

Mit dem System 100, welches eine Haar-Aufnahmevorrichtung 8 als auch ein Transportbehältnis 24 aufweist kann mit verständlicher schriftlicher und bildlicher Anleitung jede Privatperson selbständig Haare von sich oder anderen Personen entnehmen und mit einfachen Handgriffen für den Transport vorbereiten. Ferner kann auf geschultes Personal für die Haarentnahme verzichtet werden. Das Herausziehen der Haare erfolgt mittels einer Pinzette, oder indem Haare durch Rupfen mit der Hand herausgezogen werden. Im Anschluss erfolgt ein einfaches Auflegen und Positionieren der Haare auf dem Trägerplattenelement 10 und eine Befestigung der Haare 6 durch das Deckplattenelement 12. Schließlich kann der Transport über den Postweg bei Raumtemperatur stattfinden.

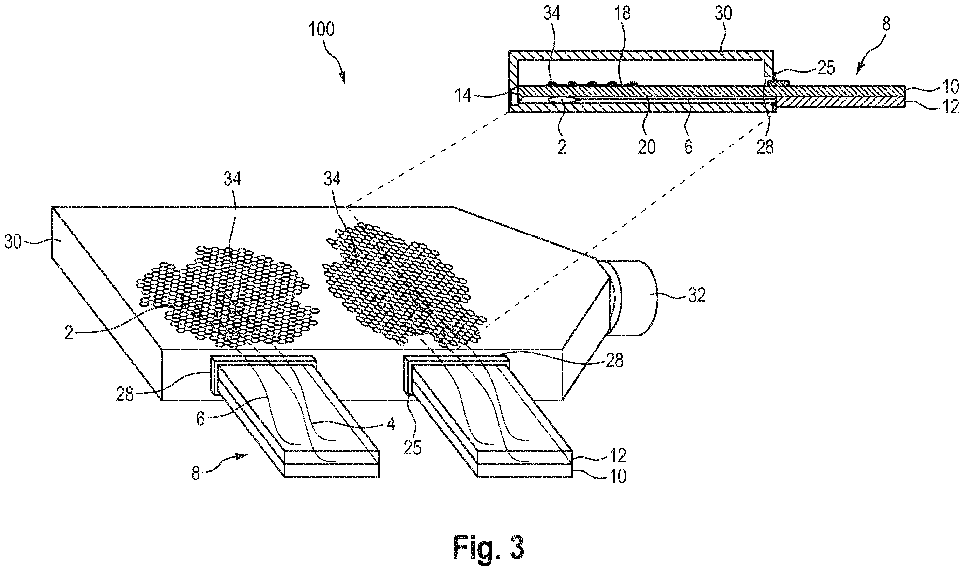

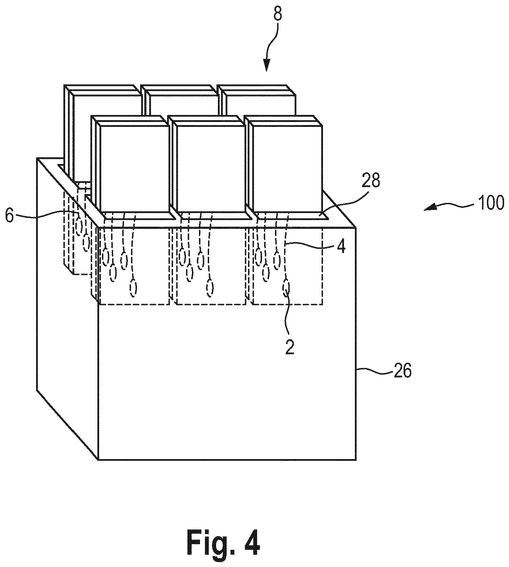

Die Haar-Aufnahmevorrichtung 8 kann nach dem Transport im Transportbehältnis 24 oder im Anschluss an die Haarentnahme in einem Kultivierungsbehältnis 30 kultiviert werden (vgl. Fig. 3) oder in einem Lagerungsbehältnis 26 (Fig. 4) eingefroren werden.

Fig. 3 lässt eine weitere Ausführungsform des Systems 100 zur Asservation von zumindest einem eine Haarwurzel 2 und einen Haarschaft 4 aufweisenden Haar 6 erkennen, wobei neben der Haar-Aufnahmevorrichtungen 8 ein Kultivierungsbehältnis 30 gezeigt ist. Neben der perspektivischen Darstellung des Systems 100 ist auch ein Querschnitt des Systems 100 in dieser Figur erkennbar. Die schematische Darstellung der Fig. 3 beinhaltet ferner die schematische Darstellung der Haar-Aufnahmevorrichtung 8 aus Fig. 1A mit den dort beschriebenen Merkmalen.

Die Haar-Aufnahmevorrichtung 8 liegt dabei zum Teil innerhalb des Kultivierungsbehältnisses 30 vor. Neben der ersten Öffnung 28 zum Einschieben einer Haar-Aufnahmevorrichtung 8 weist das Kultivierungsbehältnis 30 eine zweite Öffnung 32 auf, welche zum Austausch und/oder Zugabe von Kultivierungsmedien geeignet ist.

In Fig. 3 ist die zweite Öffnung 32 als Schraubverschluss dargestellt. Eine andere Ausführungsform der Öffnung 32 ist möglich. Ferner ist es möglich, dass das Kultivierungsbehältnis 30 mehrere Öffnungen 32 zum Austausch und/oder Zugabe von Kultivierungsmedien aufweist (nicht gezeigt). Durch das Aufweisen mehrerer Öffnungen 32 ist die Kultivierung von Keratinozyten 34 im kontinuierlichen Durchfluss möglich. Dies bietet den Vorteil, dass die Kultivierung automatisiert stattfinden kann.

Neben der Kultivierung von Keratinozyten 34 ist ferner auch die Reprogrammierung zu iPS-Zellen im Kultivierungsbehältnis möglich. Weist das Kultivierungsbehältnis 30 mehrere Öffnungen 32 zum Austausch und/oder Zugabe von Kultivierungsmedien auf, ist ferner auch eine komplett automatisierte Generierung von iPS-Zellen möglich.

Wie der Querschnitt der Ausführungsform in Fig. 3 zeigt, liegt die Haar-Aufnahmevorrichtung 8 derartig ausgerichtet vor, dass die Haar-Aufnahme-Oberfläche 20 zum Boden des Kultivierungsbehältnisses 30 zeigt und die Außen-Oberfläche 18 des Trägerplattenelements 10 dagegen in Richtung Oberseite des Kultivierungsbehältnisses 30 zeigt. Ferner ist dargestellt, dass sich die Öffnung 28 für die Haar-Aufnahmevorrichtung 8 relativ nah am Boden des Kultivierungsbehältnisses 30 befindet. In dieser Ausführungsform ist es möglich, dass das Haar 6 mit seiner Haarwurzel 2 am inneren Boden des Kultivierungsbehältnisses 30 aufliegt. Sofern der erste Abschnitt des Trägerplattenelements 10 zwischen der Haar-Aufnahme-Oberfläche 20 und der Außen-Oberfläche 18 ganz durchlässig ist, kann die Zellkultivierung der Keratinozyten 34 an der Außen-Oberfläche 18 des Trägerplattenelements 10 stattfinden.

In dieser Ausführungsform wird der Abstand zwischen der Haar-Aufnahmevorrichtung 8, insbesondere der Haar-Aufnahme-Oberfläche 20, und dem Boden des Kultivierungsbehältnisses 30 durch die Erhöhung des ersten Endes 14 definiert. In einer möglichen weiteren bevorzugten Ausführungsform, kann der Boden des Kultivierungsbehältnisses 30 eine Aussparung aufweisen, sodass die Erhöhung des ersten Endes 14 in die Einsparung passend eingelegt werden kann (nicht gezeigt). Hierdurch wird ein definierter Abstand erzielt, der die Zellkultivierung erleichtern kann.

Wie schon in Fig. 2 gezeigt, weist die Öffnung 28 für die Haar-Aufnahmevorrichtung 8 des Kultivierungsbehältnisses 30 eine Dichtung 25 auf. Diese Dichtung 25 kann das Austreten von Flüssigkeiten aus dem Kultivierungsbehältnis 30 verhindern. Neben der Dichtung 25 kann die Öffnung 28 des Kultivierungsbehältnisses 30 jede Form von Abdichtung aufweisen, die ein Auslaufen des Kultivierungsmediums oder anderen Flüssigkeiten verhindern kann.

Ferner kann in einer weiteren Ausführungsform (nicht gezeigt) die Öffnung 28 durch einen Verschluss, beispielsweise durch ein Gummielement oder Plastikdeckel, verschlossen werden. Dies hat den Vorteil, dass die Haar-Aufnahmevorrichtung nach ausreichendem Auswachsen der Keratinozyten entfernt werden kann. Hierdurch kann das Kultivierungsgefäß sicher verschlossen werden, sodass ein Auslaufen der Kultivierungsmedien verhindert werden kann. Die zuvor entnommene Haar-Aufnahmevorrichtung kann anschließend beispielsweise eingefroren oder in einem weiteren Kultivierungsbehältnis weiterverwendet werden. Die Keratinozyten dagegen können bis zur Konfluenz weiter proliferieren.

In Fig. 3 weist das Kultivierungsbehältnis 30 zwei Haar-Aufnahmevorrichtungen 8 auf. Ferner ist es möglich, dass das Kultivierungsbehältnis 30 eine oder mehrere Haar-Aufnahmevorrichtungen 8 aufweist. Diese Haar-Aufnahmevorrichtungen 8 können nebeneinander oder übereinander eingeschoben sein, wobei eine nebeneinander anliegende Anreihung bevorzugt ist.

In dem Kultivierungsbehältnis 30 in Figur 3, welche mehrere Öffnungen 28 für Haar-Aufnahmevorrichtungen 8 aufweist, ist es möglich, Kultivierungen mehrerer Haarproben gleichzeitig durchzuführen. Dies kann zum einen den zeitlichen Arbeitsaufwand sowie Materialkosten vermindern.

In Fig. 4 ist eine weitere Ausführungsform des Systems 100 zur Asservation von zumindest einem eine Haarwurzel 2 und einen Haarschaft 4 aufweisenden Haar 6 gezeigt, wobei neben der Haar-Aufnahmevorrichtung 8 ein Lagerungsbehältnis 26 gezeigt ist. Die schematische Darstellung der Fig. 4 beinhaltet ferner die schematische Darstellung der Haar-Aufnahmevorrichtung 8 aus Fig. 1A mit den dort beschriebenen Merkmalen.

In Fig. 4 ist zu erkennen, dass mehrere Haar-Aufnahmevorrichtungen 8 nebeneinander als auch hintereinander angeordnet werden können. Dies ermöglicht eine kompakte Lagerung von Haarproben.

In Figur 4 ist ferner gezeigt, dass die Öffnungen 28 der Haar-Aufnahmevorrichtung 8 sowohl nebeneinander als auch hintereinander angeordnet sind. In einer weiteren Ausführungsform ist eine andere Anordnung der Öffnungen 28 möglich. Bevorzugt sind die Öffnungen derartig angeordnet, dass möglichst viele Haar-Aufnahmevorrichtungen 8 in einem Lagerungsbehältnis 26 verstaut werden können.

In Figur 4 sind die Haar-Aufnahmevorrichtungen 8 von oben in das Lagerungsbehältnis 26 gesteckt. In weiteren Ausführungsformen (nicht gezeigt) können die Haar-Aufnahmevorrichtungen 8 auch von den anderen Seiten in das Lagerungsbehältnis 26 geschoben bzw. gesteckt werden. Ferner kann das Lagerungsbehältnis erfindungsgemäß auch lediglich eine Öffnung für eine Haar-Aufnahmevorrichtungen 8 aufweisen (ähnlich wie das Transportbehältnis 24).

Das Lagern der Haare 6 findet bevorzugt bei niedrigen Temperaturen statt, sodass die Haarproben eingefroren werden können. Dafür kann das Lagerungsbehältnis 26 mit einem Kältemittel beispielsweise Isopropanol gefüllt werden, welches ein kontinuierliches Einfrieren bei ca. -90°C ermöglicht. Die Kühlung erfolgt bevorzugt mittels flüssigem Stickstoff. Ferner kann das Lagerungsbehältnis mit einem Einfriermedium gefüllt sein; beispielsweise 90% FBS, 10% DMSO. Eine Lagerung wäre hierbei in einem Gefrierschrank bei -80 - -90°C möglich.

Sofern die Lagerung bei niedrigen Temperaturen durchgeführt wird, ist das Lagerungsbehältnis bevorzugt aus gefrierresistenten Materialen hergestellt. Ferner kann das Lagerungsbehältnis eine Möglichkeit aufweisen, die das kontinuierliche Auftauen der Probe ermöglichen.

Neben der Lagerung der Haare 6 ist ferner auch die Lagerung der Keratinozyten-Kulturen möglich, sofern eine Kultivierung zuvor stattgefunden hat.

Die Behältnisse in Fig. 2 bis 4 sind bevorzugt steril, sodass eine weitere Behandlung der Haare 6 ohne Einschränkungen stattfinden kann.

## Patentansprüche

1. System (100) zur Asservation von zumindest einem eine Haarwurzel (2) und einen Haarschaft (4) aufweisenden Haar, wobei das System (100) folgendes aufweist:
eine Haar-Aufnahmevorrichtung (8), die ein Trägerplattenelement (10) zum Ablegen des Haares und ein auf das Trägerplattenelement (10) aufzubringendes Deckplattenelement (12) zum teilweisen Abdecken des Haares umfasst, wobei das Trägerplattenelement (10) ein erstes Ende, ein zweites Ende, eine Außen-Oberfläche (18) und eine Haar-Aufnahme-Oberfläche (20) aufweist, **dadurch gekennzeichnet, dass**
die Haar-Aufnahme-Oberfläche (20) einen ersten Abschnitt aufweist, der sich vom ersten Ende (14) bis zu einem mittleren Bereich der Haar-Aufnahme Oberfläche erstreckt, und einen zweiten Abschnitt, der sich von dem mittleren Bereich bis zum zweiten Ende (16) erstreckt, wobei der erste Abschnitt eine Aussparungen und/oder Vertiefungen aufweisende Struktur zur Aufnahme der Haarwurzel (2) aufweist, und einen zweiten Abschnitt zur Aufnahme des Haarschafts (4), und dass
das Deckplattenelement (12) zum Abdecken und/oder Einklemmen des Haares auf dem Trägerplattenelement (10) derart ausgebildet und dimensioniert ist, dass es nur den zweiten Abschnitt der Haar-Aufnahme-Oberfläche (20) des Trägerplattenelements (10) abdeckt.

2. System (100) nach Anspruch 1, **dadurch gekennzeichnet, dass** das Deckplattenelement (12) mittels eines Befestigungsmittels (22) auf dem Trägerplattenelement (10) befestigt wird, zum Abdecken und/oder Einklemmen von zumindest einem eine Haarwurzel (2) und einen Haarschaft (4) aufweisendem Haar (6).

3. System (100) nach einem der vorstehenden Ansprüche, **dadurch gekennzeichnet, dass** das erste Ende (14) des Trägerplattenelements (10), im Vergleich zu dem restlichen Trägerplattenelement (10), erhöht ist.

4. System (100) nach einem der Ansprüche 1 bis 3, **dadurch gekennzeichnet, dass** die Aussparungen und/oder Vertiefungen aufweisende Struktur des ersten Abschnittes des Trägerplattenelements zwischen der Haar-Aufnahme-Oberfläche (20) und der Außen-Oberfläche (18) ganz durchlässig ist.

5. System (100) nach einem der Ansprüche 1 bis 4, **dadurch gekennzeichnet, dass** es ferner ein Transportbehältnis (24) und/oder ein Lagerungsbehältnis (26) mit zumindest einer Öffnung (28) zum Einschieben zumindest einer Haar-Aufnahmevorrichtung (8) aufweist, wobei die Öffnung (28) des Transportbehältnisses (24) und des Lagerungsbehältnisses (26) im Wesentlichen eine gleiche Höhe und Breite entsprechend des ersten Endes der Haar-Aufnahme-Oberfläche (20) aufweist, dass die Haar-Aufnahmevorrichtung, mit ihrem ersten Abschnitt, zumindest teilweise in die Öffnung (28) des Transportbehältnisses (24) einschiebbar ist.

6. System (100) nach einem der Ansprüche 1 bis 4, **dadurch gekennzeichnet, dass** es ferner ein Kultivierungsbehältnis (30) mit zumindest einer ersten Öffnung (28) zum Einschieben zumindest einer Haar-Aufnahmevorrichtung (8) aufweist, wobei die erste Öffnung (28) des Kultivierungsbehältnisses (30) im Wesentlichen eine gleiche Höhe und Breite entsprechend des ersten Endes der Haar-Aufnahme-Oberfläche (20) aufweist, dass die Haar-Aufnahmevorrichtung (8) mit ihrem ersten Abschnitt zumindest teilweise in die Öffnung (28) des Kultivierungsbehältnisses (30) einschiebbar ist, sowie einer zweite Öffnung (32) zum Austausch und/oder Zugabe von Kultivierungsmedien.

7. System (100) nach einem der Ansprüche 1 bis 6, **dadurch gekennzeichnet, dass** das Trägerplattenelement (10), das Deckplattenelement (12), das Transportbehältnis (24), das Kultivierungsbehältnis (30) und/oder das Lagerungsbehältnis (26) aus einem Material ausgewählt aus der Gruppe bestehend aus Polypropylen, Polyethylen, Polysulfon, Teflon, FEP, Teflon PFA, Polystyrol, Polycarbonat, Styrol, Acrylonitril, Acryl, Glas und Mischungen davon, hergestellt sind.

8. System (100) nach einem der Ansprüche 1 bis 7, **dadurch gekennzeichnet, dass** das Kultivierungsbehältnis (30) Kultivierungsmedien ausgewählt aus der Gruppe bestehend aus Verbindungen zur Verbesserung der Zelladhäsion (Coating Substrate), Nährmedien, Keratinozytenmedien, Fibroblasten-Wachstumsfaktoren, Peptidasen, Antibiotika, Antimykotika oder Mischungen dieser, aufweist

9. System (100) nach einem der Ansprüche 1 bis 8, **dadurch gekennzeichnet, dass** das Transportbehältnis (24) feuchtigkeitsspendende Gele aufweist.

10. Verwendung des Systems (100) nach einem der Ansprüche 1 bis 9 zum Transportieren und/oder Lagern von Haarwurzel (2) und einen Haarschaft (4) aufweisendem Haar (6).

## Claims

1. System (100) for the preservation of a hair at least comprising a hair root (2) and a hair shaft (4), wherein the system (100) comprises the following
a hair receiving device (8) comprising a carrier plate element (10) for depositing the hair and a cover plate element (12) to be applied onto the carrier plate element (10) for partially covering the hair, wherein the carrier plate member (10) has a first end, a second end, an outer surface (18) and a hair receiving surface (20), **characterized in that**
the hair-receiving surface (20) has a first section extending from the first end (14) to a central region of the hair-receiving surface, and a second section extending from the central region to the second end (16), wherein the first section has a structure comprising recesses and/or depressions for receiving the hair root (2), and a second section for receiving the hair shaft (4), and **in that**
the cover plate element (12) for covering and/or clamping the hair on the carrier plate element (10) is configured and dimensioned in such a way that it covers only the second section of the hair-receiving surface (20) of the carrier plate element (10).

2. The system (100) according to claim 1, **characterized in that** the cover plate element (12) is fixed to the carrier plate element (10) by means of a fixing means (22) for covering and/or clamping a hair (6) having at least a hair root (2) and a hair shaft (4).

3. The system (100) according to one of the preceding claims, **characterized in that** the first end (14) of the carrier plate element (10) is raised in comparison to the rest of the carrier plate element (10).

4. The system (100) according to any one of claims 1 to 3, **characterized in that** the structure of the first section of the carrier plate element between the hair-receiving surface (20) and the outer surface (18), having recesses and/or depressions, is completely permeable.

5. The system (100) according to any one of claims 1 to 4, **characterized in that** it further comprises a transport container (24) and/or a storage container (26) with at least one opening (28) for insertion of at least one hair-receiving device (8), wherein the opening (28) of the transport container (24) and the storage container (26) has substantially the same height and width corresponding to the first end of the hair receiving surface (20), that the hair receiving device, with its first section, is at least partially insertable into the opening (28) of the transport container (24).

6. The system (100) according to any one of claims 1 to 4, **characterized in that** it further comprises a cultivation container (30) having at least one first opening (28) for insertion of at least one hair-receiving device (8), wherein the first opening (28) of the cultivation container (30) has substantially the same height and width corresponding to the first end of the hair-receiving surface (20), that the hair-receiving device (8) with its first section is at least partially insertable into the opening (28) of the cultivation container (30), as well as a second opening (32) for exchanging and/or adding cultivation media.

7. The system (100) according to one of claims 1 to 6, **characterized in that** the carrier plate element (10), the cover plate element (12), the transport container (24), the cultivation container (30) and/or the storage container (26) are made of a material selected from the group consisting of polypropylene, polyethylene, polysulfone, teflon, FEP, teflon PFA, polystyrene, polycarbonate, styrene, acrylonitrile, acrylic, glass and mixtures thereof.

8. The system (100) according to any one of claims 1 to 7, **characterized in that** the cultivation container (30) comprises cultivation media selected from the group consisting of compounds for improving the cell adhesion (coating substrates), nutrient media, keratinocyte media, fibroblast growth factors, peptidases, antibiotics, antifungals or mixtures thereof.

9. The system (100) according to one of claims 1 to 8, **characterized in that** the transport container (24) comprises moisturizing gels.

10. Use of the system (100) according to one of claims 1 to 9 for transporting and/or storing a hair (6) comprising a hair root (2) and a hair shaft (4).

## Revendications

1. Système (100) destiné à la conservation d'au moins un cheveu comportant une racine de cheveu (2) et une tige de cheveu (4), le système (100) comportant :
un dispositif de réception de cheveux (8) qui comprend un élément formant plaque de support (10) destiné à déposer les cheveux et un élément formant plaque de recouvrement (12) destiné à être appliqué sur l'élément formant plaque de support (10) et à recouvrir partiellement les cheveux, l'élément formant plaque de support (10) comportant une première extrémité, une deuxième extrémité, une surface extérieure (18) et une surface de réception de cheveux (20), **caractérisé en ce que** la surface de réception de cheveux (20) comporte une première portion qui s'étend de la première extrémité (14) à une zone médiane de la surface de réception de cheveux, et une deuxième portion qui s'étend de la région médiane à la deuxième extrémité (16), la première portion comportant une structure pourvue d'évidements et/ou de creux destinés à recevoir la racine de cheveu (2), et la deuxième portion étant destinée à recevoir la tige de cheveu (4), et **en ce que** l'élément formant plaque de recouvrement (12) destiné à recouvrir et/ou serrer les cheveux sur l'élément formant plaque de support (10) est conçu et dimensionné de manière à ne recouvrir que la deuxième portion de la surface de réception de cheveux (20) de l'élément formant plaque de support (10).

2. Système (100) selon la revendication 1, **caractérisé en ce que** l'élément formant plaque de recouvrement (12) est fixé sur l'élément formant plaque de support (10) à l'aide d'un moyen de fixation (22) afin de recouvrir et/ou serrer au moins un cheveu (6) comportant une racine de cheveu (2) et une tige de cheveu (4).

3. Système (100) selon l'une des revendications précédentes, **caractérisé en ce que** la première extrémité (14) de l'élément formant plaque de support (10) est rehaussée par rapport au reste de l'élément formant plaque de support (10).

4. Système (100) selon l'une des revendications 1 à 3, **caractérisé en ce que** la structure, pourvue d'évidements et/ou de creux, de la première portion de l'élément formant plaque de support entre la surface de réception de cheveux (20) et la surface extérieure (18) est entièrement perméable.

5. Système (100) selon l'une des revendications 1 à 4, **caractérisé en ce qu'**il comprend en outre un conteneur de transport (24) et/ou un conteneur de stockage (26) comportant au moins une ouverture (28) destinée à l'insertion d'au moins un dispositif de réception de cheveu (8), l'ouverture (28) du conteneur de transport (24) et du conteneur de stockage (26) ayant sensiblement la même hauteur et la même largeur correspondant à la première extrémité de la surface de réception de cheveux (20) que le dispositif de réception de cheveux, sa première portion pouvant être insérée au moins partiellement dans l'ouverture (28) du conteneur de transport (24).

6. Système (100) selon l'une des revendications 1 à 4, **caractérisé en ce qu'**il comprend en outre un récipient de culture (30) pourvu d'au moins une première ouverture (28) destinée à l'insertion d'au moins un dispositif de réception de cheveux (8), la première ouverture (28) du récipient de culture (30) ayant sensiblement la même hauteur et la même largeur correspondant à la première extrémité de la surface de réception de cheveux (20) que le dispositif de réception de cheveux (8), sa première portion pouvant être insérée au moins partiellement dans l'ouverture (28) du récipient de culture (30), ainsi que d'une deuxième ouverture (32) destinée à l'échange et/ou l'ajout de milieux de culture.

7. Système (100) selon l'une des revendications 1 à 6, **caractérisé en ce que** l'élément formant plaque de support (10), l'élément formant plaque de recouvrement (12), le conteneur de transport (24), le conteneur de culture (30) et/ou le conteneur de stockage (26) sont fabriqués à partir d'un matériau choisi dans le groupe comprenant le polypropylène, le polyéthylène, le polysulfone, le Téflon, le FEP, le Téflon PFA, le polystyrène, le polycarbonate, le styrène, l'acrylonitrile, l'acrylique, le verre et leurs mélanges.

8. Système (100) selon l'une des revendications 1 à 7, **caractérisé en ce que** le récipient de culture (30) comportant des milieux de culture choisis dans le groupe comprenant des composés destinés à améliorer l'adhérence cellulaire (substrats de revêtement), des milieux nutritifs, des milieux kératinocytes, des facteurs de croissance de fibroblastes, des peptidases, des antibiotiques, des agents antifongiques ou leurs mélanges.

9. Système (100) selon l'une des revendications 1 à 8, **caractérisé en ce que** le conteneur de transport (24) comporte des gels hydratants.

10. Utilisation du système (100) selon l'une des revendications 1 à 9 pour le transport et/ou le stockage de cheveux (6) comportant une racine de cheveu (2) et une tige de cheveu (4).
